(19) **Europäisches Patentamt European Patent Office Office européen des brevets**

(11) **EP 4 691 340 A1**

(12) **EUROPEAN PATENT APPLICATION**
published in accordance with Art. 153(4) EPC

(43) Date of publication:
**11.02.2026 Bulletin 2026/07**

(21) Application number: **24784201.6**

(22) Date of filing: **29.03.2024**

(51) International Patent Classification (IPC):
$A61B\ 1/07^{(2006.01)}$       $G06T\ 5/00^{(2024.01)}$
$A61B\ 1/00^{(2006.01)}$       $G06T\ 7/66^{(2017.01)}$
$G06T\ 7/90^{(2017.01)}$       $G06T\ 17/20^{(2006.01)}$

(86) International application number:
**PCT/CN2024/085076**

(87) International publication number:
**WO 2024/208117 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.04.2023 CN 202310347299**

(71) Applicant: **Wuxi Hisky Medical Technologies Co., Ltd.**
**Wuxi, Jiangsu 214000 (CN)**

(72) Inventors:
• **BAI, Di**
  **Wuxi, Jiangsu 214000 (CN)**
• **HE, Qiong**
  **Wuxi, Jiangsu 214000 (CN)**
• **SHAO, Jinhua**
  **Wuxi, Jiangsu 214000 (CN)**
• **SUN, Jin**
  **Wuxi, Jiangsu 214000 (CN)**

(74) Representative: **Elzaburu S.L.P.**
**Paseo de la Castellana 259C**
**Torre de Cristal, planta 28**
**28046 Madrid (ES)**

(54) **REAL-TIME CORRECTION METHOD AND APPARATUS FOR OPTICAL-FIBER IMAGE, AND STORAGE MEDIUM AND ELECTRONIC DEVICE**

(57)    Disclosed in the present application are a real-time correction method and apparatus for an optical fiber image, and a storage medium and an electronic device. The method includes: receiving an original signal of an optical fiber image, where the original signal is an optical signal directly outputted by an optical fiber; calculating a real-time correction coefficient according to optical fiber pairs formed by optical fiber centers of the optical fiber, where the optical fiber includes a plurality of fiber bundles, each of which corresponds to an optical fiber center; and performing a real-time correction on the original signal according to the real-time correction coefficient to determine a corrected signal. This solves the problem in the related art of there being a deviation between an optical fiber image and an original image due to noise.

```
┌─────────────────────────────────────────┐
│ Receiving an original signal of an       │
│ optical fiber image, where the original  │──⌇ S101
│ signal is an optical signal directly     │
│ outputted from the optical fiber         │
└─────────────────────────────────────────┘
                    │
                    ▼
┌─────────────────────────────────────────┐
│ Calculating a real-time correction       │
│ coefficient according to optical fiber   │
│ pairs formed by optical fiber centers    │──⌇ S102
│ of the optical fiber, where the optical  │
│ fiber includes a plurality of fiber      │
│ bundles, each of which corresponds to    │
│ an optical fiber center                  │
└─────────────────────────────────────────┘
                    │
                    ▼
┌─────────────────────────────────────────┐
│ Performing a real-time correction on     │
│ the original signal according to the     │──⌇ S103
│ real-time correction coefficient, to     │
│ determine a corrected signal             │
└─────────────────────────────────────────┘
```

FIG. 1

EP 4 691 340 A1

## Description

This application claims priority to the Chinese patent application No. 202310347299.7, filed with the China National Intellectual Property Administration on April 3, 2023, entitled "Real-time Correction Method and Apparatus for Optical-fiber Image, and Storage Medium and Electronic Device", the entire content of which is incorporated herein by reference.

## TECHNICAL FIELD

The present invention relates to the field of optical fiber imaging, and in particular, to a real-time correction method and apparatus for an optical fiber image, and a storage medium and an electronic device.

## BACKGROUND

Micro-endoscopes are extensively applied in medical observation and examination. Both optical fibers and galvanometer are essential components of micro-endoscopes, where a single optical fiber typically is composed of tens of thousands of optical fiber units, and generally the center position of each optical fiber unit is fixed. In actual use, due to limitations in the scanning accuracy of the galvanometer and bending deformation of the optical fiber during use, it is impossible to ensure that each scanning occurs at an exact identical position when the end face is fixed. Consequently, as time elapses, both the scanning position of the galvanometer and the position of the optical fiber will change, and, correspondingly, the center positions of individual optical fiber units within the single optical fiber also change. This phenomenon is referred to as "optical fiber drift".

To overcome optical fiber drift, it is necessary to perform a real-time correction on the optical fiber image according to the image outputted by the optical fiber, followed by image correction. However, when transmitting an image, the optical fiber are subject to various interferences, for example, noise, which causes the pixel signals of the acquired raw image to be inaccurate.

To date, no effective solution has been proposed for solving the technical problem in related art that there is a deviation between the optical fiber image and the original image due to noise.

## SUMMARY

A main object of the present application is to provide a real-time correction method and apparatus for an optical fiber image, and a storage medium and an electronic device so as to overcome the technical problem in related art that there is a deviation between the optical fiber image and the original image due to noise.

To achieve the above object, according to one aspect of the present application, there is provided a real-time correction method for an optical fiber image, including: receiving an original signal of an optical fiber image, where the original signal is an optical signal directly outputted by an optical fiber; calculating a real-time correction coefficient according to optical fiber pairs formed by optical fiber centers of the optical fiber, where the optical fiber includes a plurality of fiber bundles, each of which corresponds to an optical fiber center; and performing a real-time correction on the original signal according to the real-time correction coefficient, to determine a corrected signal.

The above technical contents involve: receiving an original signal of the optical fiber image, where the original signal is an optical signal directly outputted by an optical fiber; calculating a real-time correction coefficient according to optical fiber pairs formed by optical fiber centers of the optical fiber, where the optical fiber includes a plurality of fiber bundles, each of which corresponds to an optical fiber center; and performing a real-time correction on the original signal according to the real-time correction coefficient to determine a corrected signal. By determining the real-time correction coefficient using the optical fiber pairs and performing the real-time correction on the original signal of the optical fiber, the accuracy of the optical fiber image is improved, and the deviation in the optical fiber image is eliminated, thereby solving the technical problem in the related art that there is a deviation between the optical fiber image and the original image due to noise.

Optionally, calculating the real-time correction coefficient according to the optical fiber pairs formed by the optical fiber centers of optical fiber includes: determining, from the plurality of optical fiber centers of the optical fiber image, the optical fiber pairs including the optical fiber centers; calculating an optical fiber pair parameter and an optical fiber pair correlation according to optical fiber pairs in multi-frame sequential images; and calculating the real-time correction coefficient according to the optical fiber pair parameter and the optical fiber pair correlation.

Optionally, determining, from the plurality of fiber bundles of the optical fiber, the optical fiber pairs including the optical fiber centers includes: determining a plurality of adjacent optical fiber centers located within a preset range around an optical fiber center; and forming an optical fiber pair by combining the optical fiber center with each of the plurality of adjacent optical fiber centers.

Optionally, calculating the optical fiber pair parameter and the optical fiber pair correlation according to optical fiber pairs in multi-frame sequential images includes: fitting grayscale values of each optical fiber pair according to optical fiber pairs in multi-frame sequential images to determine their variation relationship curve; calculating the optical fiber pair parameter according to the variation relationship curve, where the optical fiber pair parameter includes a gain parameter and a bias parameter; and calculating the optical fiber pair correlation according to the Pearson correlation coefficient of the grayscale values of each optical fiber pair.

Optionally, calculating the real-time correction coefficient according to the optical fiber pair parameter and the optical fiber pair correlation includes: determining a weight of the optical fiber pair according to the optical fiber pair correlation; and calculating the real-time correction coefficient according to the gain parameter, the bias parameter, and the weight of the optical fiber pair, where the real-time correction coefficient includes a real-time gain coefficient and a real-time bias coefficient.

Optionally, performing the real-time correction on the original signal according to the real-time correction coefficient to determine the corrected signal includes: calculating the corrected signal by multiplying the original signal by the real-time gain coefficient and adding the real-time bias coefficient.

Optionally, prior to fitting the grayscale values of each optical fiber pair according to optical fiber pairs in multi-frame sequential images to determine their variation relationship curve, the method further includes: discarding pixel point data with zero grayscale value from the multi-frame sequential images; and discarding outlier points from the multi-frame sequential images, where the outlier points are data that are determined based on a residual median of the optical fiber pair and are located far from a position of the residual median.

To achieve the above object, according to another aspect of the present application, there is provided a real-time correction apparatus for an optical fiber image, including: a receiving module, configured to receive an original signal of an optical fiber image, where the original signal is an optical signal directly outputted by an optical fiber; a calculating module, configured to calculate a real-time correction coefficient according to optical fiber pairs formed by optical fiber centers of the optical fiber, where the optical fiber includes a plurality of fiber bundles, which include an optical fiber center; and a correcting module, configured to perform a real-time correction on the original signal according to the real-time correction coefficient, to determine a corrected signal.

To achieve the above object, according to a further aspect of the present application there is provided a computer-readable storage medium for storing a program, where the program executes any one of the real-time correction methods for the optical fiber image described above.

To achieve the above object, according to a further aspect of the present application there is provided an electronic device, including one or more processors and a memory, where the memory stores one or more programs that, when is executed by the one or more processors, cause the one or more processors to implement any one of the real-time correction methods for the optical fiber image described above.

## BRIEF DESCRIPTION OF THE DRAWINGS

FIG. 1 is a flowchart of a real-time correction method for an optical fiber image according to an embodiment of the present application.
FIG. 2 is a schematic view of a real-time correction apparatus for an optical fiber image according to an embodiment of the present application.
FIG. 3 is a schematic view of an electronic device according to an embodiment of the present application.

## DETAILED DESCRIPTION OF THE EMBODIMENTS

It should be noted that, without confliction, the embodiments and features in the embodiments in the present application can be combined with one another. The present application will be hereinafter described in detail with reference to the accompanying drawings and in conjunction with the embodiments.

To enable those skilled in the art to better understand the present application, the technical solutions in the embodiments of the present application are clearly and completely described below in conjunction with the drawings in the embodiments of the present application. Obviously, the described embodiments are only some of the embodiments of the present application, rather than all the embodiments. All other embodiments obtained by persons of ordinary skill in the art without creative effort based on the embodiments disclosed in the present application shall fall within the scope of protection of the present application.

It should be noted that the terms "first," "second," etc. used in the description, claims, and drawings of the present application are for distinguishing similar objects and do not necessarily indicate a specific sequence or chronological order. It should be understood that such data may be interchanged where appropriate in order to describe the embodiments of the

EP 4 691 340 A1

present application. In addition, the terms "include," "has" and any variations thereof are intended to cover non-exclusive inclusion; for example, a process, method, system, product, or device that includes a series of steps or units is not necessarily limited to those steps or units explicitly listed, but may also include other steps or units that are not expressly set forth or that are inherent to these processes, methods, products, or devices.

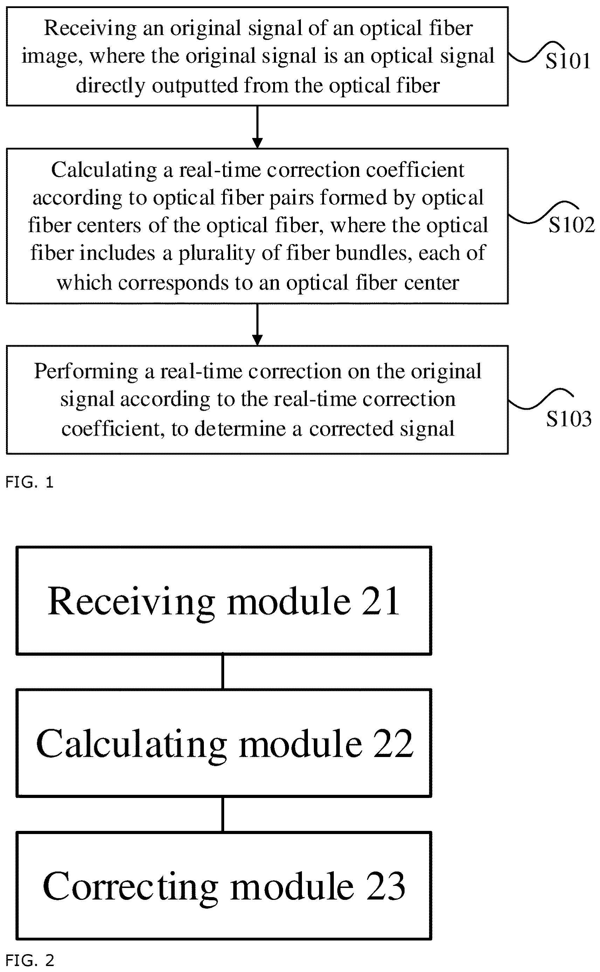

The preferred embodiment steps are now used to illustrate the present invention. FIG. 1 is a flowchart of a real-time correction method for an optical fiber image provided in an embodiment of the present application; as shown in FIG. 1, the method includes the following steps:

step S101: receiving an original signal of an optical fiber image, where the original signal is an optical signal directly outputted from the optical fiber;
step S102: calculating a real-time correction coefficient according to optical fiber pairs formed by optical fiber centers of the optical fiber, where the optical fiber includes a plurality of fiber bundles, each of which corresponds to an optical fiber center;
step S103: performing a real-time correction on the original signal according to the real-time correction coefficient, to determine a corrected signal.

The above steps involve: receiving an original signal of the optical fiber image, where the original signal is an optical signal directly outputted by an optical fiber; calculating a real-time correction coefficient according to optical fiber pairs formed by optical fiber centers of the optical fiber, where the optical fiber includes a plurality of fiber bundles, each of which corresponds to an optical fiber center; and performing a real-time correction on the original signal according to the real-time correction coefficient to determine a corrected signal. By determining the real-time correction coefficient using the optical fiber pairs and performing the real-time correction on the original signal of the optical fiber, the accuracy of the optical fiber image is improved, and the deviation in the optical fiber image is eliminated, thereby solving the technical problem in the related art that there is a deviation between the optical fiber image and the original image due to noise.

An executing entity of the above steps may be a medical endoscope apparatus, which may include a data-processing device such as processor, calculator, or controller, and is configured to perform data-processing operations of the above steps, for example steps S101 to S103.

The above medical endoscope apparatus may include an endoscope, an optical fiber, an image processor, and a display; the endoscope acquires an image, the image is transmitted through the optical fiber to the image processor for image processing, and the processed image can then be displayed by the display. The image processor may include devices with data processing capabilities, such as the above processor, calculator, controller, or server.

In view of the fact that, during transmission, the optical fiber image is subject to interference from various factors, resulting in the acquired optical fiber image signal differing from the acquired original image signal, the present embodiment provides a real-time correction method, which utilizes optical fiber pairs containing optical fiber centers within the optical fiber to calculate a real-time correction coefficient and uses this real-time correction coefficient to perform a real-time correction on the optical fiber image so that subsequent analysis and display can be carried out.

The optical fiber includes a plurality of fiber bundles, each fiber bundle having a corresponding image region in the optical fiber image. The optical fiber center is a pixel point or pixel coordinates corresponding to the center of the fiber bundle within the image region that corresponds to the center of the bundle.

It should be noted that, in order for the optical fiber centers to effectively form a plurality of optical fiber pairs, the above selected optical fiber center may be one of a plurality of optical fiber centers in the central region of the optical fiber image. The optical fiber center selected in this manner has adjacent optical fiber centers in all directions, and when forming optical fiber pairs, this optical fiber center forms optical fiber pairs with other optical fiber centers located within a preset range. The mathematical model of the connection relationship established in this way is more in line with the actual situation, and the real-time correction coefficient obtained through calculation is more accurate.

The real-time correction coefficient may include a gain parameter and a bias parameter. The corrected signal may be obtained by multiplying the original signal by the gain parameter and then adding the bias parameter.

Optionally, calculating the real-time correction coefficient according to the optical fiber pairs of the optical fiber centers of optical fiber includes: determining, from the plurality of optical fiber centers of the optical fiber image, the optical fiber pairs including the optical fiber centers; calculating an optical fiber pair parameter and an optical fiber pair correlation according to optical fiber pairs in multi-frame sequential images; and calculating the real-time correction coefficient according to the optical fiber pair parameter and the optical fiber pair correlation.

Since a plurality of optical fiber centers exist within the optical fiber image, there are numerous possible ways to form the optical fiber pairs. During analysis, it is necessary to first select one optical fiber center for analysis, to obtain the real-time correction coefficient of this optical fiber center. During correction, the image region corresponding to this optical fiber center is corrected using the real-time correction coefficient, thereby achieving correction of the entire optical fiber image.

In this embodiment, all optical fiber centers may be analyzed individually to maximize the correction effect of the

4

optical fiber image.

Optionally, determining, from the plurality of fiber bundles of the optical fiber, the optical fiber pairs including the optical fiber centers includes: determining a plurality of adjacent optical fiber centers located within a preset range around an optical fiber center; and forming an optical fiber pair by combining the optical fiber center with each of the plurality of adjacent optical fiber centers.

When selecting optical fiber pairs, a currently analyzed optical fiber center is taken as a center, and a plurality of adjacent optical fiber centers within a preset range around it are each paired with the currently analyzed optical fiber center to form optical fiber pairs. This enables that the correlation of optical fiber pairs determined by the constructed data model more accurately reflects the actual situation, thereby increasing accuracy of the correlation.

Optionally, calculating the optical fiber pair parameter and the optical fiber pair correlation according to the optical fiber pairs in multi-frame sequential images includes: fitting grayscale values of each optical fiber pair according to the optical fiber pairs in multi-frame sequential images to determine their variation relationship curve; calculating the optical fiber pair parameter according to the variation relationship curve, where the optical fiber pair parameter includes a gain parameter and a bias parameter; and calculating the optical fiber pair correlation according to Pearson correlation coefficient of the grayscale values of each optical fiber pair.

The mathematical model established through the optical fiber pairs is:

$$v_j(t) = \frac{\alpha_j}{\alpha_i}(v_i(t) - \beta_i) + \beta_j + \epsilon_{ij}(t)$$

$$v_j(t) \simeq a_{ij}v_i(t) + b_{ij}$$

upon solving, the following is obtained:

$$a_{ij} = \frac{\alpha_j}{\alpha_i}$$

$$b_{ij} = \beta_j - \frac{\alpha_j}{\alpha_i}\beta_i$$

where $u_i(t)$ denotes a grayscale value at a center position of i-th optical fiber unit in t-th frame of an optical fiber image, $u_j(t)$ denotes a grayscale value at a center position of j-th optical fiber unit in t-th frame of the optical fiber image, $\alpha_i$ denotes a gain coefficient of a grayscale value at a center position of i-th optical fiber unit, $\beta_i$ denotes a bias coefficient of a grayscale value at a center position of i-th optical fiber unit, $\alpha_j$ denotes a gain coefficient of a grayscale value at a center position of j-th optical fiber unit, $\beta_j$ denotes a bias coefficient of a grayscale value at a center position of j-th optical fiber unit, and $\epsilon_{ij}(t)$ denotes an error term between grayscale values at center positions of i-th and j-th optical fiber units in t-th frame of the optical fiber image and a fitted curve.

The Pearson correlation coefficient is used to measure the degree of correlation between $v_i$ and $v_j$, and the Pearson correlation coefficient between the two variables is defined as a ratio of covariance of the two variables to a product of their standard deviations:

$$\rho_{X,Y} = \frac{COV(X, Y)}{\sigma_X \sigma_Y}$$

where $\rho_{X,Y}$ is the correlation between the two optical fiber units $v_i$ and $v_j$ of the optical fiber pair, X is a grayscale value of $v_i$, Y is a grayscale value of $v_j$, cov is the covariance, and $\sigma_X$ and $\sigma_Y$ are the standard deviations of $v_i$ and $v_j$, respectively.

Optionally, calculating the real-time correction coefficient according to the optical fiber pair parameter and the optical fiber pair correlation includes: determining a weight of the optical fiber pair according to the optical fiber correlation; calculating the real-time correction coefficient according to the gain parameter, the bias parameter and the weight of the optical fiber pair, where the real-time correction coefficient includes a real-time gain coefficient and a real-time bias coefficient.

A corresponding weight is assigned to each optical fiber pair according to the degree of correlation of the optical fiber pairs, and the real-time correction coefficient, including the real-time gain coefficient and the real-time bias coefficient, is calculated according to the optical fiber pair coefficients. The original image is corrected in real time to obtain a true signal.

Optionally, performing the real-time correction on the original signal according to the real-time correction coefficient to determine the corrected signal includes: multiplying the original signal by the real-time gain coefficient and adding the real-time bias coefficient, to obtain the corrected signal.

Optionally, prior to fitting the grayscale values of each optical fiber pair according to optical fiber pairs in multi-frame sequential images to determine their variation relationship curve, the method further includes: discarding pixel point data with zero grayscale value from the multi-frame sequential images; and discarding outliers from the multi-frame sequential images; where the outliers are data that are determined based on a median of residuals of the optical fiber pair and are located far from a position of the median of residuals, that is, data whose distance from a position of the residual median exceeds a preset distance.

When calculating the optical fiber pair coefficients, data of pixels whose grayscale values are zero are discarded and outlier points are discarded, neither of which participates in the calculation of the optical fiber pair parameters. The method for discarding the outlier points is: setting an outlier point criterion for each optical fiber pair according to a residual median $\sigma$ of each optical fiber pair. Data of pixels whose grayscale values are zero are invalid signals, which will introduce noise when participating in the calculation of the optical fiber pair parameters, so discarding the data of pixels whose grayscale values are zero leads to more accurate image correction; when the data contain data with large errors, discarding these outlier points may reduce an influence on the fitting result, leading to more accurate image correction.

It should be noted that the steps illustrated in the flowchart of the drawings may be executed in a computer system such as a set of computer-executable instructions, and that, although a logical sequence is shown in the flowchart, in some circumstances the steps shown or described may be performed in an order different from the one presented herein.

It should be noted that the present application further provides an optional embodiment, which will be described in detail below.

This embodiment provides real-time correction method for an optical fiber image. The terms involved in this embodiment are explained below:

Optical fiber center: an optical fiber includes cladding layer and a core, the core has high transmittance, the pixel point with the maximum transmittance in each optical fiber (hexagonal structure) is regarded as the optical fiber center of this optical fiber.

Optical fiber pair: when a laser source excites an object to be detected, signals collected by optical fibers located close to one another exhibit a certain relationship; the optical fibers that exhibit such a relationship are considered an optical fiber pair.

Optical fiber pair coefficient: it is solved according to N frames of images and used to calculate the real-time correction coefficient.

Due to interference of various factors on the optical fiber image, the acquired pixel signals of an original image are inaccurate, it is a process of restoring the original true signal by a mathematical model to address the issue of inaccurate pixel signals.

$$\text{Original signal} \times \text{Real-time correction coefficient} = \text{Corrected signal}$$

Real-time correction coefficient: it is calculated according to the optical fiber pair coefficients and used to correct an image in real time.

Problems in the related art: the calculated real-time correction coefficient is inaccurate, and the image obtained after real-time correction of the original image still contains a significant noise.

The method of this embodiment is as follows: forming optical fiber pairs by pairing an optical fiber center with other optical fiber centers located within a circular neighborhood (R > 10) of the optical fiber center, to directly establish a stable connection relationship; establishing a mathematical model based on an optical fiber pair relationship established over N sequential frames of images; performing regression to solve the optical fiber pair coefficient, calculating the optical fiber pair correlation, and assigning a corresponding weight to each of the optical fiber pairs according to their correlation degree; calculating a real-time correction coefficient according to the optical fiber pair coefficient to correct an original image in real time to obtain an true signal.

Orthogonal regression is used to estimate the optical fiber pair parameters: gain $a_{ij}$ and bias $b_{ij}$

$$v_j(t) = \frac{\alpha_j}{\alpha_i}\left(v_i(t) - \beta_i\right) + \beta_j + \varepsilon_{ij}(t)$$

$$v_j(t) \simeq a_{ij} v_i(t) + b_{ij}$$

$$a_{ij} = \frac{\alpha_j}{\alpha_i}$$

$$b_{ij} = \beta_j - \frac{\alpha_j}{\alpha_i}\beta_i$$

where ui(t) denotes a grayscale value at a center position of i-th optical fiber unit in t-th frame of an optical fiber image, uj(t) denotes a grayscale value at a center position of j-th optical fiber unit in t-th frame of the optical fiber image, $\alpha i$ denotes a gain coefficient of a grayscale value at a center position of i-th optical fiber unit, $\beta i$ denotes a bias coefficient of a grayscale value at a center position of i-th optical fiber unit, $\alpha j$ denotes a gain coefficient of a grayscale value at a center position of j-th optical fiber unit, $\beta j$ denotes a bias coefficient of a grayscale value at a center position of j-th optical fiber unit, and $\varepsilon ij(t)$ denotes an error term between grayscale values at center positions of i-th and j-th optical fiber units in t-th frame of the optical fiber image and a fitted curve.

When calculating the optical fiber pair coefficients, data of pixels whose grayscale values are zero are discarded and outlier points are discarded, neither of which participates in the calculation of the optical fiber pair parameters. The method for discarding the outlier points is: setting an outlier point criterion for each optical fiber pair according to a residual median $\sigma$ of each optical fiber pair.

Advantageous effects: data of pixels whose grayscale values are zero are invalid signals, which will introduce noise when participating in the calculation of the optical fiber pair parameters, so discarding the data of pixels whose grayscale values are zero leads to more accurate image correction; when the data contain data with large errors, discarding these outlier points may reduce an influence on the fitting result, leading to more accurate image correction.

Optical fiber pair correlation: the Pearson correlation coefficient is used to measure the degree of correlation between $v_i$ and $v_j$, and the Pearson correlation coefficient between the two variables is defined as a ratio of covariance of the two variables to a product of their standard deviations:

$$\rho_{X,Y} = \frac{COV(X, Y)}{\sigma_X \sigma_Y}$$

where $\rho_{X,Y}$ is correlation between the two optical fiber units $v_i$ and $v_j$ of the optical fiber pair, X is a grayscale value of $v_i$, Y is a grayscale value of $v_j$, cov is the covariance, and $\sigma_X$ and $\sigma_Y$ are the standard deviations of $v_i$ and $v_j$, respectively.

The weight of each optical fiber pair is activated according to the optical fiber pair correlation.

The real-time correction coefficients $\alpha i$ and $\beta i$ are obtained to correct the original signal of the optical fiber image.

In related art, optical fiber centers form a triangular mesh, and two optical fiber centers on each side form one optical fiber pair; in this way, an optical fiber center only directly form fiber optic pairs with surrounding pixel points, maintaining a weak connection relationship with optical fiber centers outside a range in which the surrounding pixel points are located, and if a certain optical fiber center is an invalid signal, the overall connection relationship is affected, ultimately leading to inaccurate real-time correction coefficients.

**EP 4 691 340 A1**

In this embodiment, an optical fiber center is paired with other optical fiber centers located within its circular neighborhood (R > 10) to form optical fiber pairs. By this direct and stable pairing method of the optical fiber pairs and assigning a corresponding weight to each optical fiber pair according to their correlation degree, the real-time correction coefficients of the optical fibers are calculated. Because the mathematical model established by this method better conforms to the characteristics of optical fibers, use of the real-time correction coefficients obtained through calculation to perform the real-time correction may obtain better image quality.

FIG. 2 is a schematic diagram of a real-time correction apparatus for an optical fiber image provided by an embodiment of the present application. As shown in FIG. 2, an embodiment of the present application further provides a real-time correction apparatus for an optical fiber image. It should be noted that the real-time correction apparatus for the optical fiber image according to the embodiment of the present application may be used to perform the real-time correction method for the optical fiber image provided by the embodiments of the present application. The real-time correction apparatus for the optical fiber image provided by the embodiment of the present application will be described below. The apparatus includes: a receiving module 21, a calculating module 22, and a correcting module 23, detailed as follows.

The receiving module 21 is configured to receive an original signal of an optical fiber image, where the original signal is an optical signal directly outputted by an optical fiber; the calculating module 22 is connected to the receiving module 21 and is configured to calculate a real-time correction coefficient according to optical fiber pairs formed by optical fiber centers of the optical fiber, where the optical fiber includes a plurality of fiber bundles, each of which corresponds to an optical fiber center; the correcting module 23 is connected to the calculating module 22 and is configured to perform a real-time correction on the original signal according to the real-time correction coefficient, to determine a corrected signal.

The above real-time correction apparatus for the optical fiber image may receive an original signal of an optical fiber image, where the original signal is an optical signal directly outputted by an optical fiber; calculate a real-time correction coefficient according to optical fiber pairs formed by optical fiber centers of the optical fiber, where the optical fiber includes a plurality of fiber bundles, each of which corresponds to an optical fiber center; and perform a real-time correction on the original signal according to the real-time correction coefficient, to determine a corrected signal. By determining the real-time correction coefficient using the optical fiber pairs and performing the real-time correction on the original signal of the optical fiber, the accuracy of the optical fiber image is improved, and the deviation in the optical fiber image is eliminated, thereby solving the technical problem in the related art that there is a deviation between the optical fiber image and the original image due to noise.

The real-time correction apparatus for the optical fiber image includes a processor and a memory; the receiving module 21, the calculating module 22, the correcting module 23 and the like are all stored as program units in the memory, and the processor executes these program units stored in the memory to implement corresponding functions.

The processor includes a kernel, and the kernel retrieves the corresponding program unit from the memory. One or more kernels may be provided, and by adjusting the parameters of the kernel, the problem in the related art that a user cannot determine whether a non-original capacitive stylus of a capacitive screen is compatible with the capacitive screen in use is solved.

The memory may include, for example, a non-permanent memory, random access memory (RAM) and/or non-volatile memory in a computer-readable medium, such as read-only memory (ROM) or flash RAM, and the memory includes at least one memory chip.

An embodiment of the present invention provides a computer-readable storage medium having stored thereon a program which, when executed by a processor, implements a real-time correction method for an optical fiber image or a correction method for an optical fiber image.

An embodiment of the present invention provides a processor for executing a program, where the program, when executed, performs a real-time correction method for an optical fiber image or a correction method for an optical fiber image.

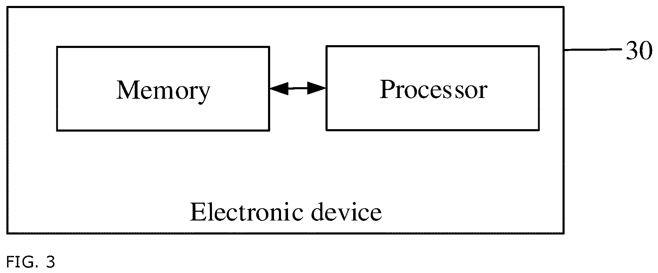

FIG. 3 is a schematic view of an electronic device provided by an embodiment of the present application. As shown in FIG. 3, an embodiment of the present application provides an electronic device 30, the device includes a processor, a memory, and a program stored on the memory and executable on the processor, the processor, when executing the program, is configured to implement the steps of the real-time correction method for an optical fiber image or the correction method for an optical fiber image.

The device herein may be a server, a PC, a PAD, a mobile phone, or the like.

The present application further provides a computer-program product which, when executed on a real-time correction apparatus for an optical fiber image, is adapted to carry out a program initialized with any one of the above method steps.

A person skilled in the art should understand that the embodiments of the present application may be provided as a method, a system, or a computer-program product. Accordingly, the present application may take the form of an entirely hardware embodiment, an entirely software embodiment, or an embodiment combining software and hardware aspects. Moreover, the present application may be embodied in the form of a computer-program product implemented on one or more computer-usable storage media (including, but not limited to, disk storage, CD-ROM, optical storage, etc.)

containing computer-usable program codes.

The present application is described with reference to flowcharts and/or block diagrams of methods, apparatus (systems), and computer-program products according to embodiments of the present application. It should be understood that each process and/or block in the flowcharts and/or block diagrams, as well as combinations of processes and/or blocks in the flowcharts and/or block diagrams, can be implemented by computer-program instructions. These computer-program commands may be provided to a general-purpose computer, a special-purpose computer, an embedded processor, or other programmable processors of the real-time correction apparatus for an optical fiber image, to produce a machine, such that the instructions executed by the computer or other programmable processors of the real-time correction apparatus for an optical fiber image create means for implementing the functions specified in one or more processes of the flowchart and/or one or more blocks of the block diagram.

These computer-program instructions may also be stored in a computer-readable memory that can direct a computer or other programmable optical fiber image real-time correction apparatus to operate in a specific manner, such that the instructions stored in the computer-readable memory produce an article of manufacture including instruction means that implements the functions specified in one or more processes of the flowchart and/or one or more blocks of the block diagram.

These computer-program instructions may also be loaded onto a computer or other programmable optical fiber image real-time correction apparatus, enabling the execution of a series of operational steps on the computer or other programmable apparatus to generate computer-implementable processing so that the instructions executable on the computer or other programmable apparatus provide steps for implementing the functions specified in one or more processes of the flowchart and/or one or more blocks of the block diagram.

In a typical configuration, a computing device includes one or more processors (CPUs), input/output interfaces, network interfaces, and internal memory.

The memory may include non-permanent memory, random access memory (RAM), and/or non-volatile memory in a computer-readable medium, such as read-only memory (ROM) or flash RAM. The memory is an example of a computer-readable medium.

A computer-readable medium includes permanent and non-permanent, removable and non-removable media, and may implement information storage by any method or technology. The information may be computer-readable instructions, data structures, program modules, or other data. Examples of computer storage media include, but are not limited to, phase-change memory (PRAM), static random-access memory (SRAM), dynamic random-access memory (DRAM), other types of random-access memory (RAM), read-only memory (ROM), electrically erasable programmable read-only memory (EEPROM), flash memory or other memory technologies, compact disc read-only memory (CD-ROM), digital versatile disc (DVD) or other optical storage, magnetic cassettes, magnetic tape storage, magnetic disk storage or other magnetic storage devices, or any other non-transmission medium that can be used to store information accessible by a computing device. As defined herein, a computer-readable medium does not include transitory media such as modulated data signals and carrier waves.

It should also be noted that the terms "include," "contain," or any other variation thereof are intended to cover non-exclusive inclusion, so that a process, method, article, or device that includes a series of elements includes not only these elements but also other elements not expressly listed, or elements inherent to such a process, method, article, or device. Without further limitation, an element defined by the phrase "including a ..." does not exclude the presence of additional identical elements in the process, method, article, or device that includes this element.

A person skilled in the art should understand that the embodiments of the present application may be provided as a method, system, or computer-program product. Therefore, the present application may take the form of an entirely hardware embodiment, an entirely software embodiment, or an embodiment combining software and hardware aspects. Moreover, the present application may be embodied in the form of a computer-program product implemented on one or more computer-usable storage media (including, but not limited to, magnetic disk storage, CD-ROM, optical storage, and the like) containing computer-usable program codes.

The above are merely embodiments of the present application and are not intended to limit the present application. For those skilled in the art, the present application may have various modifications and variations. Any modifications, equivalent substitutions, improvements, and the like made within the spirit and principles of the present application shall be included within the scope of the claims of the present application.

**Claims**

1. A real-time correction method for an optical fiber image, comprising:

   receiving an original signal of an optical fiber image, wherein the original signal is an optical signal directly outputted by an optical fiber;

calculating a real-time correction coefficient according to optical fiber pairs formed by optical fiber centers of the optical fiber, wherein the optical fiber comprises a plurality of fiber bundles, each of which corresponds to an optical fiber center; and

performing a real-time correction on the original signal according to the real-time correction coefficient to determine a corrected signal.

2. The method according to claim 1, wherein calculating the real-time correction coefficient according to optical fiber pairs formed by optical fiber centers of the optical fiber comprises:

determining, from the plurality of optical fiber centers of the optical fiber image, the optical fiber pairs comprising the optical fiber centers;

calculating an optical fiber pair parameter and an optical fiber pair correlation according to optical fiber pairs in multi-frame sequential images; and

calculating the real-time correction coefficient according to the optical fiber pair parameter and the optical fiber pair correlation.

3. The method according to claim 2, wherein determining, from the plurality of fiber bundles of the optical fiber, the optical fiber pairs comprising the optical fiber centers comprises:

determining a plurality of adjacent optical fiber centers located within a preset range around an optical fiber center; and

forming an optical fiber pair by combining the optical fiber center with each of the plurality of adjacent optical fiber centers.

4. The method according to claim 3, wherein calculating the optical fiber pair parameter and the optical fiber pair correlation according to the optical fiber pairs in multi-frame sequential images comprises:

fitting grayscale values of each optical fiber pair according to the optical fiber pairs in multi-frame sequential images to determine their variation relationship curve;

calculating the optical fiber pair parameter according to the variation relationship curve, wherein the optical fiber pair parameter comprises a gain parameter and a bias parameter; and

calculating the optical fiber pair correlation according to a Pearson correlation coefficient of the grayscale values of each optical fiber pair.

5. The method according to claim 4, wherein calculating the real-time correction coefficient according to the optical fiber pair parameter and the optical fiber pair correlation comprises:

determining a weight of the optical fiber pair according to the optical fiber pair correlation; and

calculating the real-time correction coefficient according to the gain parameter, the bias parameter, and the weight of the optical fiber pair, wherein the real-time correction coefficient comprises a real-time gain coefficient and a real-time bias coefficient.

6. The method according to claim 5, wherein performing the real-time correction on the original signal according to the real-time correction coefficient to determine the corrected signal comprises:
calculating the corrected signal by multiplying the original signal by the real-time gain coefficient and adding the real-time bias coefficient.

7. The method according to claim 4, wherein prior to fitting the grayscale values of each optical fiber pair according to optical fiber pairs in multi-frame sequential images to determine their variation relationship curve, the method further comprises:

discarding pixel point data with zero grayscale value from the multi-frame sequential images; and

discarding outlier points from the multi-frame sequential images, wherein the outlier points are data that are determined based on a residual median of the optical fiber pair and are located far from a position of the residual median.

8. A real-time correction apparatus for an optical fiber image, comprising:

a receiving module, configured to receive an original signal of an optical fiber image, wherein the original signal is an optical signal directly outputted by an optical fiber;

a calculating module, configured to calculate a real-time correction coefficient according to optical fiber pairs formed by optical fiber centers of the optical fiber, wherein the optical fiber comprises a plurality of fiber bundles, each of which corresponds to an optical fiber center; and

a correcting module, configured to perform a real-time correction on the original signal according to the real-time correction coefficient, to determine a corrected signal.

9.  A computer-readable storage medium, wherein the storage medium stores a program which, when executed, carries out the real-time correction method for an optical fiber image according to any one of claims 1 to 7.

10. An electronic device, comprising one or more processors and a memory, the memory storing one or more programs which, when executed by the one or more processors, cause the one or more processors to perform the real-time correction method for an optical fiber image according to any one of claims 1 to 7.

Receiving an original signal of an optical fiber image, where the original signal is an optical signal directly outputted from the optical fiber — S101

Calculating a real-time correction coefficient according to optical fiber pairs formed by optical fiber centers of the optical fiber, where the optical fiber includes a plurality of fiber bundles, each of which corresponds to an optical fiber center — S102

Performing a real-time correction on the original signal according to the real-time correction coefficient, to determine a corrected signal — S103

FIG. 1

Receiving module 21

Calculating module 22

Correcting module 23

FIG. 2

FIG. 3

# EP 4 691 340 A1

## INTERNATIONAL SEARCH REPORT

| International application No. |
|---|
| **PCT/CN2024/085076** |

| A. | CLASSIFICATION OF SUBJECT MATTER |
|---|---|

A61B1/07(2006.01)i; G06T5/00(2024.01)i; A61B1/00(2006.01)i; G06T7/66(2017.01)i; G06T7/90(2017.01)i; G06T17/20(2006.01)i

According to International Patent Classification (IPC) or to both national classification and IPC

| B. | FIELDS SEARCHED |
|---|---|

Minimum documentation searched (classification system followed by classification symbols)

IPC: A61B G06T

Documentation searched other than minimum documentation to the extent that such documents are included in the fields searched

Electronic data base consulted during the international search (name of data base and, where practicable, search terms used)

CNABS: CNTXT; ENTXTC; VEN; WPABS; ENTXT; CNKI; ISI Web of science: 无锡海斯凯尔医学, 苏州微景医学, 白迪, 何琼, 邵金华, 孙锦, 光纤, 漂移, 偏移, 校正, 系数, 灰度, 亮度, 增益, 偏置, 拟合, optical fiber?, optical fibre?, correct+, grayscale, coefficient, brightness, lum+, gain, offset, drift

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116584874 A (WUXI HISKY MEDICAL CO., LTD.) 15 August 2023 (2023-08-15) claims 1-10 | 1-10 |
| X | CN 113570520 A (SUZHOU WEIJING MEDICAL TECHNOLOGY CO., LTD.) 29 October 2021 (2021-10-29) description, paragraphs [0052]-[0120], and figures 1-8 | 1-10 |
| A | CN 111415312 A (SUZHOU INSTITUTE OF BIOMEDICAL ENGINEERING AND TECHNOLOGY, CHINESE ACADEMY OF SCIENCES) 14 July 2020 (2020-07-14) entire document | 1-10 |
| A | CN 113068015 A (NANJING RUIPU CHUANGKE TECHNOLOGY CO., LTD.) 02 July 2021 (2021-07-02) entire document | 1-10 |
| A | US 2010274082 A1 (FUJIFILM CORP.) 28 October 2010 (2010-10-28) entire document | 1-10 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 June 2024** | **03 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/085076**

| Patent document cited in search report | | | Publication date (day/month/year) | Patent family member(s) | Publication date (day/month/year) |
|---|---|---|---|---|---|
| CN | 116584874 | A | 15 August 2023 | None | |
| CN | 113570520 | A | 29 October 2021 | None | |
| CN | 111415312 | A | 14 July 2020 | None | |
| CN | 113068015 | A | 02 July 2021 | None | |
| US | 2010274082 | A1 | 28 October 2010 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310347299